Europäisches Patentamt

European Patent Office

Office européen des brevets

(19)

(11)  **EP 1 029 160 B1**

(12)  **EUROPÄISCHE PATENTSCHRIFT**

(45) Veröffentlichungstag und Bekanntmachung des Hinweises auf die Patenterteilung:
**09.04.2003  Patentblatt 2003/15**

(51) Int Cl.$^7$: **F01M 11/10**, G07C 5/00

(21) Anmeldenummer: **98958892.6**

(86) Internationale Anmeldenummer:
**PCT/EP98/06966**

(22) Anmeldetag: **04.11.1998**

(87) Internationale Veröffentlichungsnummer:
**WO 99/024699 (20.05.1999 Gazette 1999/20)**

(54) **VERFAHREN UND VORRICHTUNG ZUM ÜBERWACHEN UND/ODER ERMITTELN VON MOTORÖLQUALITÄT**

METHOD AND DEVICE FOR MONITORING AND/OR DETERMINING MOTOR OIL QUALITY

PROCEDE ET DISPOSITIF POUR SURVEILLER ET/OU DETERMINER LA QUALITE D'UNE HUILE DE MOTEUR

(84) Benannte Vertragsstaaten:
**DE ES FR GB IT SE**

(30) Priorität: **07.11.1997  DE 19749364**

(43) Veröffentlichungstag der Anmeldung:
**23.08.2000  Patentblatt 2000/34**

(73) Patentinhaber:
• **Volkswagen Aktiengesellschaft**
  **38436 Wolfsburg (DE)**
• **Mobil Schmierstoff Gmbh**
  **22297 Hamburg (DE)**

(72) Erfinder:
• **PICKERT, Detlef**
  **D-38448 Wolfsburg (DE)**
• **SCHUMACHER,Volker**
  **D-38524 Sassenburg (DE)**
• **SÖLTER, Harald**
  **D-38118 Braunschweig (DE)**
• **VÖLTZ, Martin**
  **D-22844 Norderstedt (DE)**

(56) Entgegenhaltungen:
| | |
|---|---|
| EP-A- 0 174 601 | DE-A- 3 228 195 |
| US-A- 4 677 847 | US-A- 5 060 156 |
| US-A- 5 604 441 | |

**Beschreibung**

Die Erfindung betrifft ein Verfahren zum Überwachen und/oder Ermitteln von Motorölqualität durch Bestimmung der Viskosität des Motoröls im Betrieb von Brennkraftmaschinen. Des weiteren betrifft die Erfindung eine Vorrichtung zur Durchführung des Verfahrens.

Eine Vielzahl bekannter Vorrichtungen, wie beispielsweise Werkzeugmaschinen oder Kraftfahrzeuge, müssen in bestimmten Intervallen gewartet werden, um einerseits die Zuverlässigkeit sicherzustellen und andererseits die Lebensdauer der Vorrichtung zu erhöhen. Das Motoröl des Motors eines Kraftfahrzeugs unterliegt einem Verschleiß und muß nach dem Erreichen eines bestimmten Verschleißgrades ausgewechselt werden, da sonst der Motor wegen unzureichender Schmierung und Kühlung Schaden nehmen würde. Die Lebensdauer eines Motoröls hängt jedoch von vielen Betriebsparametern ab, u.a. von Umgebungsfaktoren und der Fahrweise des Fahrers. Da diese nicht vorherbestimmbar sind, werden bestimmte Sicherheitsmargen angenommen und der Kfz-Hersteller gibt der Einfachheit halber feste Wartungsintervalle und Ölwechselintervalle vor, die i.a. durch feste Kilometerangaben realisiert werden und die einzuhalten sind, wenn die vom Hersteller übernommene Garantie in Kraft bleiben soll. Dies führt dazu, daß der Kfz-Halter oftmals ohne technische Notwendigkeit eine Inspektion oder einen Ölwechsel vornehmen läßt, was einen erheblichen zusätzlichen Kostenfaktor darstellt. Es wurden daher bereits frühzeitig Anstrengungen unternommen, die Ölwechselintervalle an den tatsächlichen Verschleiß des Motoröls anzupassen.

Beim gattungsgemäßen Verfahren kann beispielsweise der Verschmutzungsgrad des Motoröls in Abhängigkeit vom elektrischen Widerstand, dem Differenzdruck zwischen Auf und Abstromseite des Ölfilters, der Lichtdurchlässigkeit oder der chemischen Zusammensetzung des Motoröls direkt bestimmt werden. Nachteilig bei diesen direkten Meßmethoden ist der zusätzliche meßtechnische Aufwand, beispielsweise die Notwendigkeit zusätzlicher und spezieller Sensoren etc.. Zusätzlich zu den direkten Meßmethoden gibt es daher Verfahren, bei denen aus anderweitig bekannten Betriebsparametem des Motors bzw. des Fahrzeugs auf den Verschleißgrad des Motoröls rückgeschlossen wird.

Die EP 0 174 601 offenbart ein Wamsystem, das den Verschleiß bzw. die Alterung des Öls einer Brennkraftmaschine mißt und zur Anzeige bringt, bzw. ein Wamsignal abgibt. Dabei wird aus den Motorparametem Drehzahl, aktueller Motorbelastung und Öltemperatur eine Aussage über den Ölzustand abgeleitet und ausgegeben.

Die DE 41 31 969 zeigt eine Schmierölüberwachungseinrichtung , bei der mittels eines speziellen Sensorchips die Ölparameter Druck, Temperatur und Viskosität erfaßt werden, um daraus den Ist-Zustand des Motoröls abzuleiten. Dabei wird die Viskosität des Motoröls durch die Messung der Dielektrizitätskonstanten des Öls bei zwei verschiedenen Frequenzen kapazitiv ermittelt. Alternativ kann die Viskosität des Motoröls auch über eine Messung der Dämpfung von Schallwellen im Motoröl ermittelt werden.

Die DE 32 28 195 offenbart ein Verfahren und eine Vorrichtung zur Überwachung des Zeitpunkts des Schmierölwechsels für einen Kraftfahrzeugmotor. Wesentlicher Schritt des Verfahrens ist die Bestimmung des Anteils an Verunreinigungen im Motoröl, der auf der Grundlage von Motorbetriebsbedingungen abgeleitet werden kann, wobei der Grad der Verunreinigungen in unmittelbarer Beziehung zu der Viskosität des Motoröls steht.

Nachteilig bei den bekannten Verfahren ist, daß entweder zusätzliche Sensoren benötigt werden oder der Schluß auf den Verschleißgrad des Motoröls aus bekannten Betriebsparametem nicht die gewünschte Zuverlässigkeit besitzt und daher aus Sicherheitsgründen das Motoröl zu früh mit der damit verbundenen nachteiligen Kostenbetastung des Kfz-Halters gewechselt wird.

Der Erfindung liegt daher die Aufgabe zugrunde, ein Verfahren zu entwickeln, das eine einfache und genaue Überwachung bzw. Ermittlung der Motorölqualität eines Kfz-Motors ermöglicht. Des weiteren ist es Aufgabe, eine Vorrichtung zur Durchführung des Verfahrens zu schaffen.

Diese Aufgabe wird durch die Merkmale der Ansprüche 1, 6 und 13 gelöst. Bevorzugte Ausgestaltungen der Erfindung sind Gegenstand der Unteransprüche.

Diese Aufgabe wird erfindungsgemäß dadurch gelöst, daß Änderungen in der Viskosität des Öls in Abhängigkeit von Motortemperatur und Motorreibmement ermittelt und ausgewertet werden. Das erfindungsgemäße Verfahren ermöglicht ohne zusätzliche Meßinstrumente in zuverlässiger Weise die Ermittlung der Viskositätsänderung des Motoröls, die ihrerseits für die Überwachung der Motorölqualität herangezogen wird. Durch die Kenntnis der Motorölqualität ist ein Ölwechsel nur dann erforderlich, wenn das Motoröl tatsächlich verschlissen ist.

Bei einer bevorzugten Ausführungsform des Verfahrens wird das Motorreibmoment aus der Bestimmung des Anlassermoments abgeleitet. Somit wird eine einfache Bestimmung des Motorreibmoments erreicht.

Bei einer weiteren vorteilhaften Form der Erfindung wird das Anlaßmoment bei Kenntnis der Anlassercharakteristik aus der vom Anlasser aufgenommenen elektrischen Leistung während des Anlaßvorgangs bestimmt. Dieses Verfahren ist besonders einfach, weil die Stromaufnahme im wesentlichen der Batteriebelastung entspricht und somit leicht zu ermitteln ist. DIe Abhängigkeit der Stromaufnahme von der Motorölqualität ist somit einfach zur Qualitätsermittlung bzw. Auswertung der ermittelten Daten heranzuziehen.

Vorteilhafterweise wird eine Viskositätsänderung nur berücksichtigt, wenn der Wert (IstWert) bei gleicher Temperatur außerhalb eines Bereichs von -15% bis +50% von einem vorgegebenen Viskositätswert liegt. Dadurch wird verhindert, daß bereits leichte Schwankungen der Viskosität durch verschiedene Randparamter zu einer Anzeige für einen erforderlichen Ölwechsel führen. Es wird sichergestellt, daß lediglich signifikante Veränderungen bei der Überwachung berücksichtigt werden und Folgeaktionen erst zum richtigen Zeitpunkt ausgelöst werden.

Die der Erfindung zugrundeliegende Aufgabe wird weiterhin gemäß Anspruch 6 durch ein Verfahren zur Bestimmung der Viskosität des Motoröls einer Brennkraftmaschine, gelöst. Dadurch, daß die Viskosität des Motoröls aus dem Motorreibmoment ermittelt wird, wobei das Motorreibmoment aus in einem Motorsteuergerät vorhandenen Daten bestimmt wird, ist auf einfache Weise zu ermitteln, wann ein Ölwechsel durchgeführt werden muß.

Vorzugsweise werden bei einem Ottomotor die folgenden Motordaten zur Bestimmung des Motorreibmoments verwendet: Einspritzzeit und/oder Drosselklappenstellung zur Bestimmung des erzeugten Motormoments, ein Signal des Kupplungsschalters, das anzeigt, ob ein Moment an den Antriebsstrang abgegeben wird, das Lastsignal des Generators zur Bestimmung des Generatorantriebsmoments, und Signale über den Betriebszustand etwaiger weiterer direkt vom Motor angetriebener Nebenaggregate. Auf diese Weise ist eine zuverlässige Ermittlung der Motorölqualität gewährleistet.

Bei einem Dieselmotor werden die folgenden Motordaten zur Bestimmung des Reibungsmoments verwendet: Ein Signal des Kupplungsschalters, das anzeigt, ob ein Moment an den Antriebsstrang abgegeben wird, das Lastsignal des Generators als Maß für die vom Generator erzeugte elektrische Energie, die Motordrehzahl, die Einspritzmenge, die Motortemperatur und die Umgebungstemperatur. Dies ermöglicht eine zuverlässige Ermittlung der Motorölqualität.

In einen weiterer Ausführungsform wird während des Anlaßvorgangs die Zeit vom Start bis zum Erreichen der Startabwurfdrehzahl gemessen so daß mit der Kenntnis der in dieser Zeit eingespritzten konstanten Kraftstoffmenge aus der gemessenen Zeit auf die Größe des Reibmoments des Motors geschlossen werden kann, wordurch eine zuverlässige und genaue Ermittlung der Motorölqualität gewährleistet ist.

Die Aufgabe wird weiterhin erfindungsgemäß durch eine Vorrichtung zur Durchführung des Verfahrens gelöst. Zur Bestimmung der Viskosität weist sie eine Steuereinheit sowie mindestens eine Speichereinheit auf. Eine derartige Vorrichtung ermöglicht die Ermittlung der Motorölqualität in einfacher Weise, da keine zusätzlichen Meßmittel erforderlich sind.

Weitere Ausführungsformen der Erfindung ergeben sich aus den Unteransprüchen und der Beschreibung. Bevorzugte Ausführungsformen sind nachfolgend anhand der Zeichnungen näher erläutert:

Fig. 1    zeigt ein Schemabild der Bestimmung der Ölviskosität bei einem Dieselmotor, und

Fig. 2    zeigt ein Schemabild der Bestimmung der Viskosität aus der vom Anlasser aufgenommenen elektrischen Leistung.

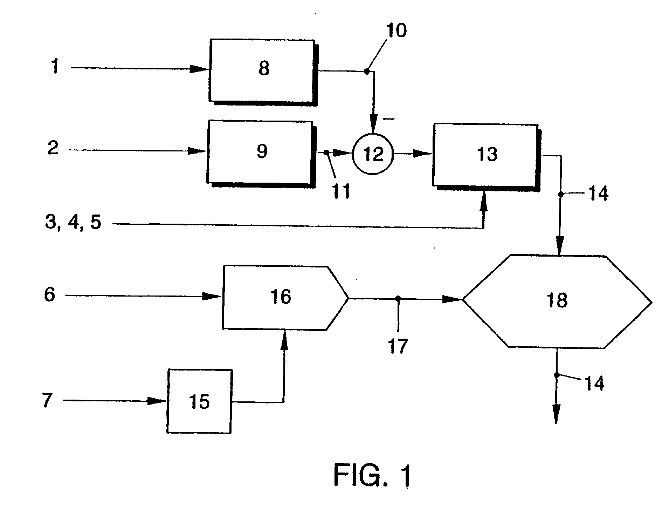

Das der Fig. 1 zugrundeliegende Berechnungsverfahren basiert auf dem Momentengleichgewicht des ausgekuppelten Motors im Leerlauf. In diesem Betriebspunkt sind die meisten Größen konstant, so daß ihr Einfluß auf das erzeugte Motormoment in Kennfeldern, vorzugsweise in der Form von Lookup-Tabellen, abgelegt werden kann.

Die stationäre Momentenbilanz eines Motors lautet:

$$M_{Motor} = M_{Kupplung} + M_{Nebenaggregate} + M_{Reibung} + M_{Verdichtung} \tag{1}$$

$$\text{mit } M_{Nebenaggregate} = M_{Wasserpumpe} + M_{Ölpumpe} + M_{Generator}, \tag{2}$$

falls sonst keine Nebenaggregate angeschlossen sind.

Unter Leerlaufbedingungen, d.h. ausgekuppelt, gelten die folgenden Gleichungen:

$$M_{Kupplung} = 0 \text{ (etwaige Last ist abgetrennt)}, \tag{3}$$

$$N_{Motor} = \text{konstant} \Rightarrow dN/dt = 0 \text{ (Leerlaufdrehzahl ist geregelt)}, \tag{4}$$

$$M_{Wasserpumpe} = \text{konstant}, \tag{5}$$

$$M_{Generator} = f(P_{elektrisch}),$$

(Generatormoment ist eine Funktion der elektrischen Leistung)   (6)

$$M_{Verdichtung} = f(T_{Motor}, T_{Umgebung}),$$

(Verdichtungsmoment des Motors ist eine Funktion der Motor- und der Umgebungstemperatur)   (7)

$$M_{Reibung} + M_{Ölpumpe} = f(v_{Öl}, T_{Motor}, T_{Umgebung}),$$   (8)

und

$$M_{Motor\ Leerlauf} = f(v_{Öl}, T_{Motor}, T_{Umgebung}) + M_{Generator} = f(m_E),$$

(das Motormoment im Leerlauf ist eine Funktion der eingespritzten Kraftstoffmenge)   (9)

daraus ergibt sich für die Viskosität unter der Annahme der obigen Gleichungen (3) - (9) im Leerlaufbetrieb:

$$v_{Öl} = f(M_{Motor\ Leenauf} - M_{Generator}, T_{Motor}).$$   (10)

Bezogen auf eine Referenztemperatur $T_0$ des Öls, die beispielsweise 40°C oder 100°C betragen kann, ergibt sich:

$$V_{Öl\ T0} = f(v_{ÖL}, T_{Öl} / T_0)$$   (11).

Dabei wurden die folgenden Definitionen getroffen:

M = Moment. N = Drehzahl, T = Temperatur, P = Leistung,
$m_E$ = Einspritzmenge, v = Viskosität.

Die verwendeten Indizes sind selbsterklärend.

Die Figur 1 zeigt das Blockschaltbild für diese Berechnung am Beispiel eines Dieselmotors. Als Eingangsgrößen stehen das Generatorlastsignal 1 als Maß für die vom Generator erzeugte elektrische Leistung $P_{elektr}$, die Einspritzmenge $m_E$ 2, Motortemperatur $T_{Motor}$ 3, Umgebungstemperatur $T_{Umgebung}$ 4 und Öltemperatur $T_{Öl}$ 5, sowie das Kupplungssignal 6, das anzeigt, ob ausgekuppelt ist oder nicht, und die Motordrehzahl N 7 zur Verfügung. Über ein in einer ersten Kennlinieneinheit 8 abgelegtes erstes Kennlinienfeld wird das Generatorlastsignal 1 in das entsprechende Generatormoment 10 umgerechnet. In gleicher Weise wird die Einspritzmenge 2 über ein in einer zweiten Kennlinieneinheit 9 abgelegtes zweites Kennlinienfeld in das Leerlaufmoment des Motors $M_{Motor\ Leerlauf}$ 11 umgerechnet. Durch Differenzbildung der beiden so erhaltenen Momente 10 und 11 in einem Subtrahierer 12 ergibt sich das gesuchte Reibmoment der Gleichung (9), das eine Funktion der Ölviskosität ist. Über ein in einer dritten Kennlinieneinheit 13 abgelegtes drittes Kennlinienfeld wird daraus unter Berücksichtigung der Motortemperatur 3, der Umgebungstemperatur 4 und der Öltemperatur 5, die Ölviskosität 14 bezogen auf die Bezugstemperatur entsprechend der Gleichungen (10) und (11) bestimmt. Die in den Kennlinieneinheiten 8, 9 und 13 abgelegten Kennlinien bzw. Kennlinienfelder sind motorspezifisch und werden empirisch bestimmt. Da die Drehzahl des Motors vom Leerlaufregler konstant gehalten wird, braucht sie in den nichtlinearen Kennlinienfunktionen der Kennlinieneinheiten 8, 9, 13 nicht berücksichtigt zu werden. Die Motordrehzahl 7 wird in einem Differentiator 15 nach der Zeit abgeleitet. Das Motordrehzahldifferential wird zusammen mit dem Kupplungssignal 6 in einem logischen "UND"-Gatter 16 zu einem Betriebspunktsignal 17 verknüpft. In einem weiteren Logikgatter oder Betriebspunktgatter 18 legt das Betriebspunktsignal 17 des "Und"-Gatters 16 fest, ob die bestimmte normierte Ölviskosität 14 gültig ist oder nicht, d.h. ob die Randbedingungen (3) und (4) der Gleichungen (10) und (11) erfüllt sind.

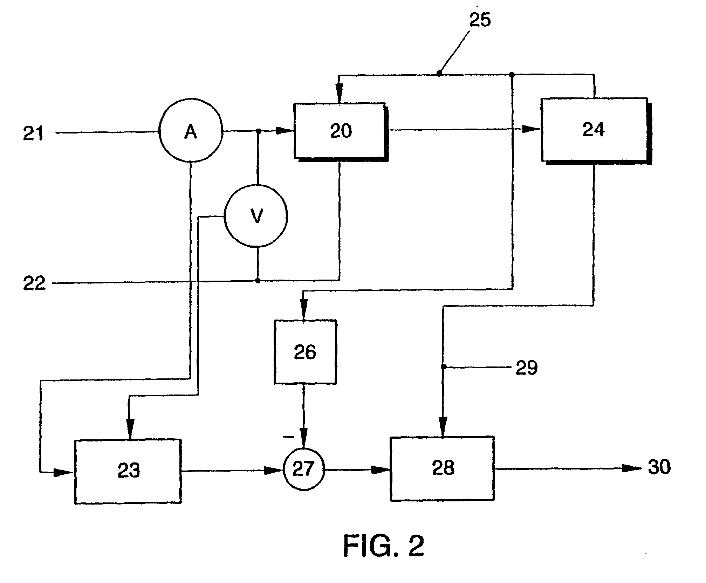

Das in der Fig. 2 dargestellte Verfahren zur Bestimmung der Ölviskosität beruht auf der Auswertung der Leistungsbilanz des Anlassvorgangs. Da hier zum einen alle Verbraucher weitgehend abgeschaltet sind und der Generator in diesem Drehzahlbereich ohnehin so gut wie keine elektrische Energie liefert, kann das Generatormoment genau wie die durch die anderen Nebenaggregate (mit Ausnahme der Ölpumpe) hervorgerufenen Lastmomente in erster Näherung als bei jedem Startfall gleich angenommen werden, wobei gleiche Umgebungsbedingungen vorausgesetzt werden. Das Motorreibmoment und die Verdichtungsarbeit können ebenfalls als Funktionen der Motortemperatur und der Zeit angesetzt werden. Da das Motorreibmoment und insbesondere das Antriebsmoment der Ölpumpe darüberhinaus wesentlich von der Viskosität des Motoröls abhängen, kann diese bei einem Startvorgang aus den Abweichungen der Anlasserleistung zu bekannten Referenzzuständen bestimmt werden.

Dargestellt in der Fig. 2 ist ein Anlasser 20, der über Leitungen 21 und 22 während des Anlassvorgangs mit Strom beaufschlagt wird. Der entsprechende Strom und die Spannung werden über entsprechende Meßeinheiten A, V bestimmt. Aus diesen Daten wird in einer Berechnungseinheit 23 die Anlasserleistung nach

$$P_{Anlasser} = \eta_{Anlasser} * I * U \qquad (12)$$

bestimmt. Das vom Anlasser 20 erzeugte Anlassermoment wirkt auf einen Motor 24. Aus der dabei erzeugten Motordrehzahl 25 wird in einer weiteren Berechnungseinheit 26 die Beschleunigungsleistung des Motors 24 nach

$$P_{Bes} = N * \Theta * dN/dt \qquad (13)$$

berechnet. Die in einem Subtrahierer 27 ermittelte Differenz $\Delta P$ der Anlasserleistung und der Beschleunigungsleistung ist die gesuchte Reibleistung des Motors, die einem Reibmoment entspricht. In einer Kennlinieneinheit 28 wird daraus unter Berücksichtigung der Motortemperatur 29 die Ölviskosität 30 über

$$v_{ÖL} = f(\Delta P, T_{Motor}) \qquad (14)$$

bestimmt.

Die verwendeten Bezeichnungen sind wie folgt definiert:

P = Leistung, $\eta$ = Wirkungsgrad, I = Strom, U = Spannung,
N = Drehzahl, $\Theta$ = Trägheitsmoment, $\Delta P$ = Reibleistung.

In einer dritten, nicht dargestellten Ausführungsform wird während des Anlassvorgangs die Zeit vom Starten bis zum Erreichen der Startabwurfdrehzahl gemessen. Das Steuergerät des Motors spritzt während des Starts eine feste Menge Treibstoff ein, bis die Startabwurfdrehzahl erreicht ist. Dann schaltet das Steuergerät auf normale Leerlaufregelung um. Der genaue Zeitpunkt des Umschaltens hängt von der Momentenbilanz des Motors in der Startphase ab. Da der Verlauf des erzeugten Moments sich aus der Kraftstoffmenge ergibt und bekannt ist, kann aus der Zeit bis zum Erreichen der Startabwurfdrehzahl auf die Größe des Verlustmoments, d.h. des Reibmoments des Motors, geschlossen werden. In Abhängigkeit von der Zusatzlast kann damit aus Referenzversuchen auf die Viskosität des Motoröls geschlossen werden. Als Signal aus dem Motorsteuergerät kann für diese Messung das "Status-Bit-Motor-Normalbetrieb" verwendet werden. Dieses Bit ist in der Startphase "0" und wird bei Erreichen der Startabwurfdrehzahl auf "1" gesetzt. Die Startabwurfdrehzahl liegt üblicherweise bei 1200 U/min.

**BEZUGSZEICHENLISTE**

1    Generatorlastsignal
2    Einspritzmenge
3    Motortemperatur
4    Umgebungstemperatur
5    Öltemperatur
6    Kupplungssignal
7    Motordrehzahl
8    erste Kenntinieneinheit

9   zweite Kennlinieneinheit
10  Generatormoment
11  Motorleerlaufmoment
12  Subtrahierer
13  dritte Kennlinieneinheit
14  Ölviskosität
15  Differentierer
16  UND-Gatter
17  Betriebspunktsignal
18  Betriebspunkt-Gatter
20  Anlasser
21  Leitung
22  Leitung
23  Berechnungseinheit
24  Motor
25  Motordrehzahl
26  Berechnungseinheit
27  Subtrahierer
28  Kennlinieneinheit
29  Motortemperatur
30  Ölviskosität

**Patentansprüche**

1. Verfahren zum Überwachen und/oder Ermitteln der Motorölqualität durch Bestimmung der Viskosität des Motoröls im Betrieb von Brennkraftmaschinen, insbesondere Fahrzeugmaschinen, **dadurch gekennzeichnet, daß** Änderungen in der Viskosität des Öls in Abhängigkeit von Motortemperatur ($T_{Motor}$) und Motorreibmoment ermittelt und ausgewertet werden.

2. Verfahren nach Anspruch 1, **dadurch gekennzeichnet, daß** das Reibmoment des Motors aus der Bestimmung des Anlassermoments abgeleitet wird.

3. Verfahren nach Anspruch 1 oder 2, **dadurch gekennzeichnet, daß** das Anlassermoment bei Kenntnis der Anlassercharakteristik aus der vom Anlasser aufgenommenen elektrischen Leistung während des Anlaßvorgangs bestimmt wird.

4. Verfahren nach Anspruch 2, **dadurch gekennzeichnet, daß** das Reibmoment des Motors aus der aufgenommenen Motorbeschleunigungsteistung abgeleitet wird.

5. Verfahren nach einem der vorangegangenen Ansprüche, **dadurch gekennzeichnet, daß** eine Viskositätsänderung lediglich dann berücksichtigt wird, wenn der Wert (IstWert) bei gleicher Temperatur außerhalb eines Bereichs von -15% bis +50% von einem vorgegebenen Viskositätswert liegt.

6. Verfahren zur Bestimmung der Viskosität des Motoröls einer Brennkraftmaschine, **dadurch gekennzeichnet, daß** die Viskosität des Motoröls aus dem Motorreibmoment ermittelt wird.

7. Verfahren nach Anspruch 6, **dadurch gekennzeichnet, daß** das Motorreibmoment aus im Motorsteuergerät vorhandenen Motordaten bestimmt wird.

8. Verfahren nach Anspruch 7, **dadurch gekennzeichnet, daß** bei einem Ottomotor die folgenden Motordaten zur Bestimmung des Motorreibmoments verwendet werden:

Einspritzzeit und/oder Drosselklappenstellung zur Bestimmung des erzeugten Motormoments,
ein Signal, das anzeigt, ob ein Moment an den Antriebsstrang abgegeben wird,
das Lastsignal des Generators zur Bestimmung des Generatorantriebsmoments,
und Signale über den Betriebszustand etwaiger weiterer direkt vom Motor angetriebener Nebenaggregate.

9. Verfahren nach Anspruch 7, **dadurch gekennzeichnet, daß** bei einem Dieselmotor die folgenden Motordaten zur Bestimmung des Reibungsmoments verwendet werden:

ein Signal, das anzeigt, ob ein Moment an den Antriebsstrang abgegeben wird,
das Lastsignal des Generators als Maß für die vom Generator erzeugte elektrische Energie,
die Motordrehzahl,
die Einspritzmenge,
die Motortemperatur, und
die Umgebungstemperatur.

10. Verfahren nach einem der Ansprüche 7 bis 9, **dadurch gekennzeichnet, daß** das Reibmoment des Motors aus der Bestimmung des Anlassermoments und der aufgenommenen Motorbeschleunigungsleistung abgeleitet wird.

11. Verfahren nach Anspruch 10, **dadurch gekennzeichnet, daß** das Anlassermoment sich bei Kenntnis der Anlassercharakteristik aus der vom Anlasser aufgenommenen elektrischen Leistung während des Anlassvorgangs bestimmen läßt.

12. Verfahren nach Anspruch 10, **dadurch gekennzeichnet, daß** während des Vorgangs des Anlassens die Zeit vom Start bis zum Erreichen der Startabwurfdrehzahl gemessen wird, so daß mit der Kenntnis der in dieser Zeit eingespritzten konstanten Kraftstoffmenge aus der gemessenen Zeit auch die Größe des Reibmoments des Motors geschlossen werden kann.

13. Vorrichtung zur Durchführung der Verfahren nach einem der Ansprüche 1 bis 12, mit einer Steuereinheit sowie mindestens einer Speichereinheit zur Bestimmung einer Ölviskosität aus einem aus Motordaten bestimmten Motorreibmoment unter Verwendung motorspezifischer Kennlinien bzw. Kennlinienfeldern, die das Motorreibmoment und die Ölviskosität miteinander verknüpfen.

14. Vorrichtung nach Anspruch 13, **dadurch gekennzeichnet, daß** die motorspezifischen Kennlinien bzw. Kennlinienfelder in Form von Lookup-Tabellen vorliegen.

## Claims

1. Method for the monitoring and/or determination of engine-oil quality by the determination of the viscosity of the engine oil during the operation of internal combustion engines, in particular vehicle engines, **characterized in that** changes in the viscosity of the oil as a function of the engine temperature ($T_{Engine}$) and engine moment of friction are determined and evaluated.

2. Method according to Claim 1, **characterized in that** the moment of friction of the engine is derived from the determination of the starter moment.

3. Method according to Claim 1 or 2, **characterized in that**, with a knowledge of the starter characteristic, the starter moment is determined from the electrical power consumption of the starter during the starting operation.

4. Method according to Claim 2, **characterized in that** the moment of friction of the engine is derived from the engine acceleration power consumption.

5. Method according to one of the preceding claims, **characterized in that** a change in viscosity is taken into account only when, for the same temperature, the value (actual value) lies outside a range of -15% to +50% of a predetermined viscosity value.

6. Method for determining the viscosity of the engine oil of an internal combustion engine, **characterized in that** the viscosity of the engine oil is determined from the engine moment of friction.

7. Method according to Claim 6, **characterized in that** the engine moment of friction is determined from engine data present in the engine control unit.

8. Method according to Claim 7, **characterized in that**, in a petrol engine, the following engine data are used for

**EP 1 029 160 B1**

determining the engine moment of friction:

  injection time and/or throttle-valve position for determining the engine torque generated,
  a signal which indicates whether a torque is transferred to the drive train,
  the load signal of the alternator for determining the alternator drive torque,
  and signals relating to the operating state of any further secondary assemblies driven directly by the engine.

9. Method according to Claim 7, **characterized in that**, in a diesel engine, the following engine data are used for determining the moment of friction:

  a signal which indicates whether a torque is transferred to the drive train,
  the load signal of the alternator as a measure of the electric energy generated by the alternator,
  the engine rotational speed,
  the injection quantity,
  the engine temperature, and
  the ambient temperature.

10. Method according to one of Claims 7 to 9, **characterized in that** the moment of friction of the engine is derived from the determination of the starter moment and of the engine acceleration power consumption.

11. Method according to Claim 10, **characterized in that**, with a knowledge of the starter characteristic, the starter moment can be determined from the electrical power consumption of the starter during the starting operation.

12. Method according to Claim 10, **characterized in that**, during the starting operation, the time from the start until the starter-shedding rotational speed is reached is measured, so that, with a knowledge of the constant fuel quantity injected within this time, the size of the moment of friction of the engine can also be concluded from the measured time.

13. Device for carrying out the method according to one of Claims 1 to 12, with a control unit and with at least one storage unit for determining an oil viscosity from an engine moment of friction determined from engine data, using engine-specific characteristic curves or characteristic maps which interlink the engine moment of friction and the oil viscosity.

14. Device according to Claim 13, **characterized in that** the engine-specific characteristic curves or characteristic maps are in the form of look-up tables.

**Revendications**

1. Procédé pour surveiller et/ou déterminer la qualité de l'huile du moteur en déterminant la viscosité de l'huile du moteur au cours du fonctionnement de moteurs à combustion interne, en particulier de moteurs d'automobiles, **caractérisé en ce que** des variations de la viscosité de l'huile en fonction de la température du moteur ($T_{moteur}$) et du couple de friction du moteur sont déterminées et analysées.

2. Procédé selon la revendication 1, **caractérisé en ce que** le couple de friction du moteur est dérivé de la détermination du couple du démarreur.

3. Procédé selon la revendication 1 ou 2, **caractérisé en ce que** le couple du démarreur est déterminé pendant l'opération de démarrage lorsque l'on connaît la caractéristique du démarreur à partir de la puissance électrique consommé par le démarreur.

4. Procédé selon la revendication 2, **caractérisé en ce que** le couple de friction du moteur est dérivé de la puissance d'accélération du moteur reçue.

5. Procédé selon l'une quelconque des revendications précédentes, **caractérisé en ce qu'**une variation de viscosité est seulement prise en compte lorsque la valeur (valeur instantanée) est, pour une même température, en dehors d'une plage de -15% à +50% d'une valeur de viscosité prédéfinie.

8

6. Procédé pour déterminer la viscosité de l'huile du moteur d'un moteur à combustion interne, **caractérisé en ce que** la viscosité de l'huile du moteur est déterminée à partir du couple de friction du moteur.

7. Procédé selon la revendication 6, **caractérisé en ce que** le couple de friction du moteur est déterminé à partir des données relatives au moteur existant dans l'appareil de commande du moteur.

8. Procédé selon la revendication 7, **caractérisé en ce que,** dans un moteur à allumage par étincelles, les données de moteur suivantes sont utilisées pour déterminer le couple de friction du moteur :

   durée d'allumage et/ou position de l'étrangleur pour déterminer le couple produit,

   un signal qui indique si un couple est produit au niveau de la chaîne de transmission,

   le signal de charge du générateur pour déterminer le couple d'entraînement du générateur, et des signaux concernant l'état de fonctionnement d'unités auxiliaires supplémentaires éventuelles entraînées directement par le moteur.

9. Procédé selon la revendication 7, **caractérisé en ce que,** dans un moteur diesel, les données de moteur suivantes sont utilisées pour déterminer le couple de friction :

   un signal qui indique si un couple est produit au niveau de la chaîne de transmission,

   le signal de charge du générateur servant de mesure pour l'énergie électrique produite par le générateur,

   la vitesse de rotation du moteur,

   la quantité d'injection,

   la température du moteur, et

   la température de l'environnement.

10. Procédé selon l'une quelconque des revendications 7 à 9, **caractérisé en ce que** le couple de friction du moteur est dérivé de la détermination du couple du démarreur et de la puissance d'accélération du moteur reçue.

11. Procédé selon la revendication 10, **caractérisé en ce que** le couple du démarreur peut être déterminé, lorsqu'on connaît la caractéristique du démarreur, à partir de la puissance électrique reçue par le démarreur pendant l'opération de démarrage.

12. Procédé selon la revendication 10, **caractérisé en ce que,** pendant l'opération de démarrage, on mesure l'intervalle de temps entre le démarrage et l'obtention de la vitesse de rotation de déconnexion du démarreur, de sorte que si l'on connaît la quantité de carburant constante injectée pendant ce temps, on peut à partir du temps mesurée également en conclure l'amplitude du couple de friction du moteur.

13. Dispositif pour mettre en oeuvre le procédé selon l'une quelconque des revendications 1 à 12, comprenant une unité de commande ainsi qu'au moins une unité de mémoire pour déterminer la viscosité d'une huile à partir d'un couple de friction du moteur déterminé à partir de données de moteur en utilisant des caractéristiques ou des champs caractéristiques spécifiques au moteur, qui lient l'un à l'autre le couple de friction du moteur et la viscosité de l'huile.

14. Dispositif selon la revendication 13, **caractérisé en ce que** les caractéristiques ou les champs caractéristiques spécifiques au moteur se présente sous la forme de tables à consulter.

FIG. 1

FIG. 2